# EUROPEAN PATENT SPECIFICATION

(11) **EP 2 111 208 B1**
(45) Date of publication and mention of the grant of the patent: **14.02.2018**
(21) Application number: 08707433.2
(22) Date of filing: 31.01.2008
(51) Int. Cl.: A61H 36/00

(54) **METHOD AND DEVICE FOR TENDING OR TREATING BODY TISSUE OR FOR SHAPING THE FIGURE**
VERFAHREN UND VORRICHTUNG ZUR PFLEGE ODER BEHANDLUNG VON KÖRPERGEWEBE ODER ZUR FORMUNG DER FIGUR
PROCÉDÉ ET DISPOSITIF POUR TENDRE OU TRAITER UN TISSU CORPOREL OU POUR FAÇONNER LE VISAGE

(30) Priority: 01.02.2007 US 898859 P
(43) Date of publication of application: 28.10.2009
(73) Proprietor: MRN-medical research network GmbH, 1010 Wien (AT)
(72) Inventor: MILLET, Oswald, A-1060 Vienna (AT)
(74) Representative: Frei Patent Attorneys
(86) International application number: PCT/EP2008/000744
(87) International publication number: WO 2008/092671

(56) References cited:
- EP-A- 1 479 363
- WO-A-03/028599
- DE-A1- 2 657 344
- DE-C1- 19 743 064

## Description

### FIELD OF THE INVENTION

The invention is the field of tending and treating body tissue, especially sub-skin connective tissue, and of shaping the figure. It particularly relates to a method of treating the human body with the aim to strengthen the connective tissue, to thighten the skin, to shape the figure and to improve or eliminate cellulitis.

### BACKGROUND OF THE INVENTION

Figure control and weight problems have been treated with different wrapping techniques for at least 20 years. These techniques are partially successful, but are not efficient in activating the metabolism. Further, the wrapping is labor-intensive, and therefore the treatment in cosmetic studios and fitness studios is rather costly. In the self-treatment version ("wrap yourself'), it is laborious, complicated and time consuming.

Even more popular for loosing weight, tightening tissue and achieving cosmetically beneficial effects is sports. Some findings suggest that catabolism of fat, however, only takes place where the connective tissue is well supplied with blood. At the same time, the skin and sub-skin connective tissue in the problematic zones is cooled by sports activities. The reason for this is that the muscles during sports activities are supplied with more blood, and this is compensated for by less blood flowing into the surrounding connective tissue. The connective tissue cools. As a result, the person does not lose weight in the zone she/he thinks of as problematic but in other zones. The result may be cosmetically unfavorable.
In classical wrap technics a foil is wraped around a piece of closing as shown e.g. in DE 19743064.

Similar considerations hold for the treatment of cellulite and loose connective tissue.

### SUMMARY OF THE INVENTION

It is an object of the present invention to provide a method and a device which alleviate the above-mentioned problems. Especially, the method should allow for an efficient fat catabolism in zones considered problematic, such as the sub-skin connective tissue.

It is another object of the present invention to provide a method and a device for causing an enhanced blood circulation in problematic zones of the body, especially during sports activities.

It is a further object to provide a cosmetical method and a device which provide for an efficient tightening of the skin tissue and connective tissue and to provide optimal premises for a well directed catabolism of fat.

It is yet another object to provide a method and a device for specifically enhancing the lymphatic flow.

These objects and others are achieved by the features of the independent method claim 8. As an alternative to instructing the person to exercise, the method may include the step of instructing the person to rest for some time, i.e. to wait for some time substantially passively, for example lying, sitting, standing, passively moving (motorized movement) and/or slowly walking. The watertight foil is a separate element to be got on separately and before the wrapping by an elastic element.

The covering with watertight foil and the wrapping the body part in the elastic element may be done actively on the user or may be done by instructing the user to do it.

The elastic element causes, during the exercising, compression and movement of the body to interact in a positive manner, so that for a positive effect no other - passive - stimulation of the body by massaging or vibration etc. is necessary. In a way, the compressive, elastic element in conjunction with the foil underneath simulates a healthy, tight connective tissue even if the connective tissue of the user is not tight. For the method according to the invention, it is important that the foil element is underneath the elastic element. Only by this is the foil element directly pressed onto the body and prevents sweat from being able to move away from the body and, for example, cool or even evaporate there. Also, the elastic element due to this approach does not get wet.

Preferably, an effective substance is applied to the body part prior to wrapping the body part in the foil.

The sport the user may exercise may include an indoor activity, such as a group training in a fitness center, a weight training, Pilates, treadmill, cross trainer or elliptical trainer, training with any other fitness equipment or gymnastics, including passive gymnastics. It may in addition or as an alternative include outdoor training, such as nordic walking, power walking, jogging, roller skating, cross-country skiing, ice-skating, cycling, etc. This list may be supplemented arbitrarily, since the method according to the invention is compatible with almost every sports activity, as long as it does not take place underwater.

In any case, the exercising is preferably an active exercising, where the user actively makes movements. This is in contrast to passive exercising, where the user stands, sits or lies on an electrically powered device that moves (for example by vibration) body parts or where the user is massaged. It has been found that active training is especially suited for the method according to the invention, especially if muscles in body parts wrapped by the device foil element and the elastic element according to the invention are active. The muscle pump (i.e. the effect of the muscle contraction) presses against a compression wall from an inside causing a slight pressure rise in the tissue. This effect of pressing against a compression wall furthers the absorption of the effective substance(s) and stimulates the blood circulation. Also, the stimulation reaches the smooth muscle fibers around the lymphatic vessels and stimulates them. The smooth muscle fibers as a consequence contract the lymphatic vessels and cause a lymph drainage. Of course, the method also causes the user to heavily sweat.

By the lymphatic flow, excess water is drained from the connective tissue. This causes the path from the capillary to the cell to be shortened, whereby cell supply is optimized. This, in combination with enhanced blood circulation, is a prerequisite for healthy, tight sub-skin connective tissue. Due to the watertight (and possibly also airtight) foil directly atop the skin, the skin absorbs the effective substance(s) intensely and most effectively.

So far, elastic body wraps have been considered to be effective only when in direct contact to the skin. The invention provides a new approach by adding a watertight foil directly atop the skin, beneath the skin and the elastic element. An enhanced moisturizing effect and the mentioned improved absorption of an effective substance are two advantages. Further advantages are most practical: The elastic element can have a specific shape adapted to the body part - for example it can be trousers - and nevertheless it is easy to put on and is not drained in sweat after the exercising. Especially, if the foil and the elastic element each are a corresponding piece of clothing - such as a trouser, a trouser leg, a jacket, a suit, a stocking, etc., the handling is strongly improved compared to prior art "winding" methods of wrapping a body. Whereas wrapping up a body by winding a foil around it is cumbersome, whereas due to the approach according to the invention, a loose foil piece of clothing can be easily put on, and the elastic piece of clothing is also easy to put on, because it glides on the foil. Only due to this, the putting on of the device by the user her- or himself becomes possible.

Also, wound foil easily slips and thus makes sports activities impossible, whereas the combination of a foil piece of clothing and an elastic piece of clothing holds well also during sports activities. Further, conventional wound foils tend to constrict the skin at certain places, whereas a piece of clothing does not.

A device according to the invention comprises the features of claim 1. Thus, both, the foil and the elastic element are shaped to cover a specific part of the user's body - in contrast to a foil be wound around the body. By this, the body is covered only by one layer of the foil and by one layer of the elastic element, and there are no bothering overlappings between neigbouring windings. The elastic element is separate from the watertight foil. This device has the special advantage of being easy to put on. Further, the foil need not be stretchable.

The watertight foil is a "foil item of clothing" and has a shape adapted to the body part it is intended to be on, for example a trouser-like shape, the shape of a stocking, of a glove, a sleeve, a jacket, or a shape adapted to a person's fundament, etc..

Especially preferred is a shape ("suit") substantially covering a large part of the body (except the feet and the head, and possibly but not necessarily except the shoulders, the arms and/or the breast). The shape of the foil suit may for example comprise an approximately cylindrical section for the upper body and two cylindrical sections projecting therefrom and of comparably reduced diameter for covering the legs.
The foil may for example be a plastic or cellophane foil, such as a transparent or intransparent plastic foil. As an alternative, the foil can be a metal clad, for example Aluminum clad or possibly Copper clad plastic foil. Such a clad foil features the special advantage the it is reflecting for Infrared radiation originating from the body.
Also the elastic element may have a corresponding shape and be a "elastic item of clothing". The elastic element, in a not stretched state, is preferably smaller than the foil and is smaller than the body part to be wrapped.
A special embodiment is that of a shape ("elastic suit") that covers and compresses, both, the legs (at least from the knees upwards, preferably from the calves upwards, the lower body and the torso up to the breast or up to below the bosom - for example like a for example sleeveless jumpsuit. In addition to an elastic suit, the elastic element may include a further, separate element for the region above the hips, much like a broad, elastic belt. This is especially advantageous for users with weak connective tissue in the belly region.

According to an example not claimed, the watertight foil and the elastic element may together form a common clothing element. Then, the watertight foil needs to be stretchable, too.

The device may further comprise an effective substance, for example a cosmetically effective substance or an agent for stimulating the blood circulation and/or stimulating the Lymph flow.

Such an effective substance may especially be or include a moisturising or cosmetically active substance. It may further be or include a therapeutically effective agent.

The - optional - effective substance or substances may be present as gels, emulsions, creams, oils, lotions, ointments etc. and have a potentially broad combination of contained substances. Possible tissue- and skin-caring contents are known from cosmetics. They may contain water, oils and/or alcohols and at least some of the group of extracts from ginkgo leaves, ginseng roots, macadamia, Malva sylvestris, mint, balm, marigold, algae, olives, avocado, horse chestnut, bladderwrack, stinging nettle, horsetail, jojoba oil, maize oil, almond oil, wheat germ oil, etc. and further natural ingredients as well as synthetic active ingredients.

### BRIEF DESCRIPTION OF THE DRAWINGS

In the following, preferred embodiments of the invention will be described referring to schematical drawings. In the drawings:
- Fig. la shows a watertight foil element, namely watertight foil trousers of a first embodiment of the invention;
- Fig. 1b shows an elastic element, namely, elastic trousers, of the first embodiment of the invention;
- Fig. 2a shows a watertight foil element, namely a watertight foil stocking of a second embodiment of the invention;
- Fig. 2b shows an elastic element, namely an elastic stocking, of the second embodiment of the invention;
- Fig. 3 depicts a container of an effective substance;
- Fig. 4a schematically shows how tissue portions of the body can be caused to deflect during sports activities;
- Fig. 4b shows how a device according to the invention can prevent such deflections;
- Fig. 5 very schematically shows a device according to the invention in contact with a body part;
- Fig. 6 depicts a flowchart of a method according to the invention;
- Figs. 7a-7c show a foil element and an elastic element, respectively, of a further embodiment of the invention; and
- Figs. 8a and 8b show a foil element and an elastic element, respectively, of an even further embodiment of the invention.

### DESCRIPTION OF THE PREFERRED EMBODIMENTS

The device according to the invention comprises a foil element that is essentially watertight and is adapted to be brought into contact with a body part. The foil element may be made of a plastic foil, which may be essentially transparent or may be colored or at least partially intransparent. For example, it may be made of a polyalkene foil (such as a polyethylene foil or polypropylene foil), or a PVC foil, or a polystyrene or polyester foil or acrylic foil etc.. As an alternative, it may be made of a cellophane foil. As yet another alternative, it may be made of a metal clad foil such as a medal clad plastic foil.

Preferably, the foil is not only watertight but also airtight.

Preferably, the foil is very thin. Compared to prior art devices (sweat pants for example) it is not necessary that the foil be mechanically robust. On the contrary: it has been found that comparably thin, flexible foils are advantageous in combination with the elastic element enveloping it. In this way, the effect may be optimized as the foil fits closely to the skin and in addition is agreeable to the user. The foil element also need not be stretchable. Preferably, the thickness of the foil - for example being a polyethylene foil or of an other foil material - , therefore is as small as 50 µm or less, especially preferred 30 µm or less, for example at most 20 µm.

The elastic element is made of a material with substantial elasticity. For example, it may be such as to be able to be stretched, along one direction, to at least 110% or at least 120% or 130% or 150% of its original size, or even to at least 180% of its original size. For example, it may be a tissue including a substantial portion of elastane (Spandex), and possibly including other materials such as cotton, or it may be made of other stretchable materials.

Preferably, either the foil element or the elastic element or both is/are be pre-shaped for the body part. For example the elastic element may be formed as elastic trousers or as an elastic stocking.

As a less preferred alternative, at least one of the foil and of the elastic element may be in an arbitrary shape (for example in the shape of a long coiled strip) and have to be wound around the tissue.

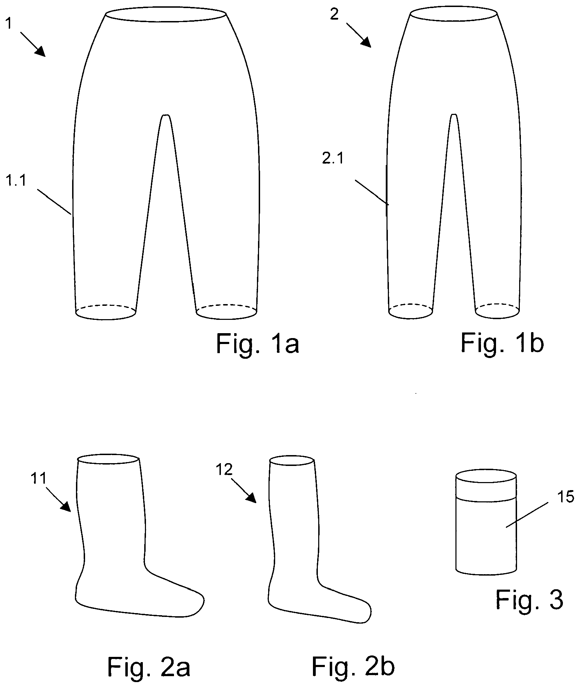

The foil trousers 1 depicted in **Fig. 1a** are adapted to fit only loosely or be too large when worn by the user. The foil trouser legs 1.1 have a diameter that is significantly greater than the diameter of the user's leg. The elastic trousers 2 shown in **Fig. 1b****,** however, are dimensioned to fit very tightly, thus to be stretched when worn by the user. The diameter of the elastic trousers' legs is therefore, when the trouser material is not stretched, smaller than the diameter of the user's leg.

In addition, the device may further comprise a belt element being a broad elastic strap for fixing the foil trouser around the waist and/or for achieving an additional compression in a belly region (see also figures described below).

Similarly, the foil stocking 11 in **Fig. 2a** is adapted to cover the user's foot only loosely. The elastic stocking 12 of **Fig. 2b****,** on the other hand, dimensioned to fit very tightly, thus to be stretched when worn by the user.

A device according to the invention may further include an effective substance of the kind mentioned above. **Fig. 3** depicts a substance as a lotion in an appropriate container 15. Of course, also different substances may be applied, for example one after an other. This may include the application of a starter substance first and than an effective substance, and/or the application of yet another substance after the elastic and foil elements have been removed.

On top of the elastic element, the user may wear additional clothing, such as a tracksuit.

After the (potential) application of the active substance and after the getting on of the foil element and the elastic element, the user is instructed to exercise or to rest.

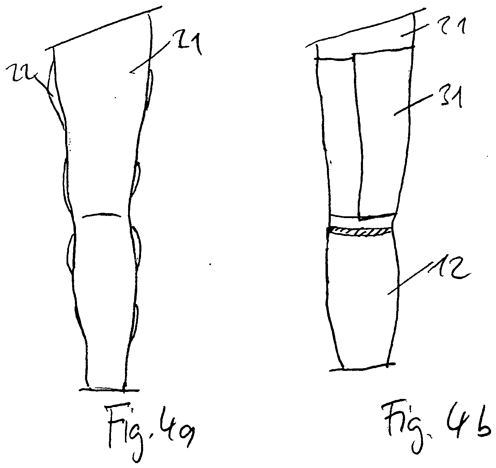

**Figs. 4a** and **4b** very schematically depict the massaging effect of the invention. During sports activities, the tissue under normal circumstances deflects. According deflections 22 of tissue of a leg 21 are illustrated in Fig. 4a. The elastic element of the device according to the invention prevents such deflections. Instead, by means of the support provided by the elastic element, at places where the tissue would otherwise deflect, a slight pressure impinges on the tissue.

In the example of Fig. 4b, for illustration purposes, two elastic elements are present. On the lower leg, the user wears an elastic stocking 12 wrapping the (not visible) watertight foil. On the thigh, the elastic element is a tightly mounted elastic sleeve 31, for example with Velcro fastener (hook and loop fastener).

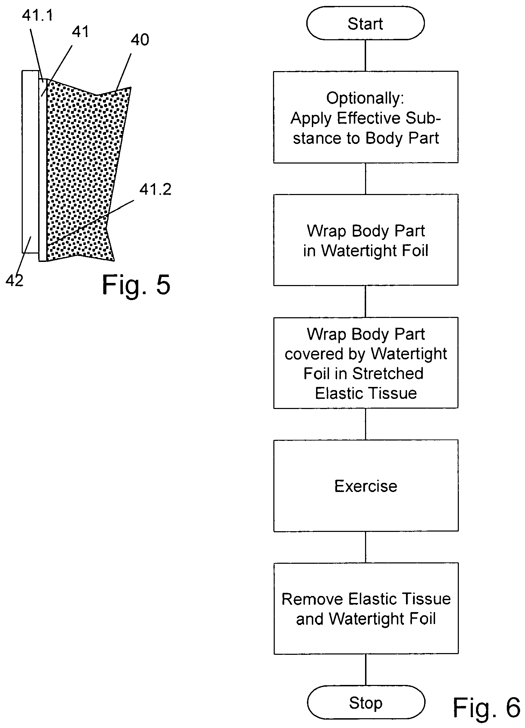

**Fig. 5** depicts a watertight foil 41 covered by the elastic element 42 adjacent body tissue 40. The watertight foil 41 comprises a plastic layer 41.1 clad by a metal layer 41.2. This special embodiment ensures that infrared radiation from the body is reflected back, so that the warming effect is even enhanced. Of course, the metal layer need not be in direct contact with the skin as illustrated. Rather, the metal layer may also be the outer layer of the foil layers.

Depending on the choice of material, the foil may be reflecting for at least a part of the IR radiation also absent any metallic coating.

**Fig. 6****,** illustrates a flowchart of a method according to the invention.

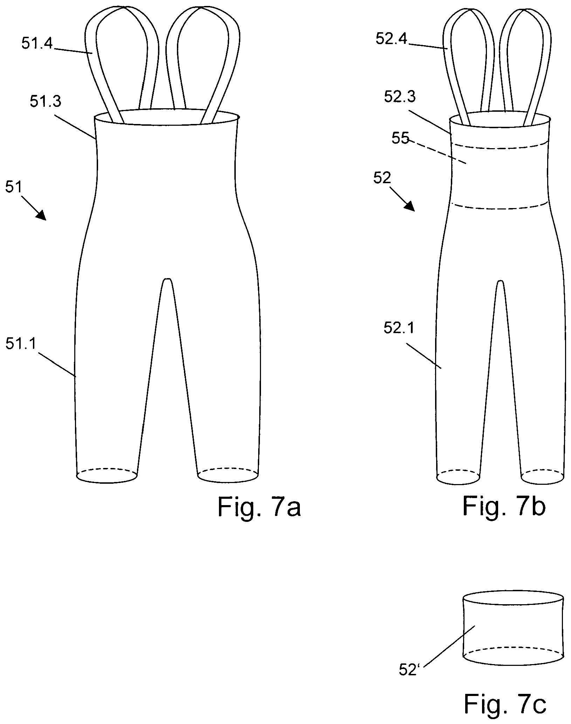

**Figs. 7a-7c** show a further, preferred embodiment of the invention. This special, preferred embodiment is characterized in that both, the legs (at least from the knees upwards, preferably at least from the calves upwards), and the abdomen at least up to the lower breast region are covered by the foil element 51 and the elastic item of clothing 52. Next to an upper approximately cylindrical section (it may be waisted as illustrated) forming an abdomen covering portion 51.3, 52.3 and two cylindrical sections projecting therefrom forming leg covering portions 51.1, 52.1, the foil and elastic items of clothing, respectively, also comprise optional suspender portions 51.4, 52.4. In addition, the elastic element comprises a separate piece, namely a waist belt 52' for enforcing the compressing action in the region above the hips. The region where the waist belt is to be worn - preferably on top of the elastic suit 52, is denoted by 55 in Fig. 7b.

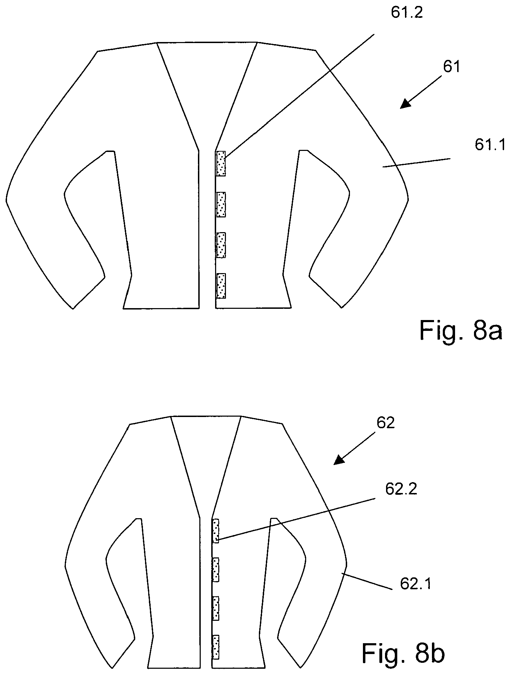

**Figs. 8a and 8b** show a further embodiment of the invention, namely a foil jacket 61, and an elastic jacket 62, respectively. The jackets are one example of pieces of clothing also at least partially covering the user's arms by comprising sleeves 61.1, 62.1. Instead, the foil and the elastic element may comprise separate sleeves, pullover-shaped items, a full body suit also covering the arms etc. In the depicted embodiment, both, the foil jacket 61 and the elastic jacket 62 each comprise velcro fasteners 61.2, 62.2.

The elastic element and/or the foil may in addition or as an alternative include or consist of other items of clothing, such as sleeves, pull-overs, full-body suits etc. Generally, the foil element (especially, the foil item of clothing) is designed to cover a somewhat larger portion of the user's body than the elastic element (the elastic item of clothing), so that the elastic element may not get into direct contact with the user's skin.

Whereas preferably the foil element and the elastic element - as in the illustrated embodiments - essentially cover the same parts of the body, this need not be the case. For example, in an alternative embodiment, the foil suit may cover the entire body except the hands, feet, neck and head, whereas the elastic element may include different garments, such as elastic trousers, an elastic shirt, and/or elastic sleeves etc.

## Claims

1. A device for at least one of figure shaping and of tending human tissue and of treating human tissue of a body part of a user's body, the device comprising:
- a watertight foil element shaped to cover a specific part of the user's body; and
- an elastic element
wherein the elastic element is shaped to envelope the watertight foil element when it is wrapped around the body part and to thereby being stretched and
wherein the elastic element is separate from the watertight foil element **characterized in that** watertight foil element is a foil item of clothing.

2. The device according to claim 1, wherein the elastic element forms at least one elastic item of clothing, the foil item of clothing covering at least all parts of the user's body that are covered by the elastic item of clothing.

3. The device according to claim 2, wherein both, the foil item of clothing and the elastic item of clothing are shaped to cover at least the thighs and the abdomen of a human body.

4. The device according any one of the previous claims, wherein the elastic element is shaped to be one of elastic trousers, of an elastic stocking, of elastic shorts, of an elastic tube to be put around a part of a leg, of an elastic sleeve to be put around a part of an arm, of an elastic jacket, of an elastic pullover, and of an elastic suit.

5. The device according to any one of the previous claims further comprising an effective substance to be applied to the skin of the user prior to putting on the watertight foil element.

6. The device according to any one of the previous claims, wherein a thickness of the foil element is not more than 50 µm, preferably not more than 30 µm, for example not more than 20 µm.

7. The device according to any one of the previous claims, wherein the elastic element is made of an elastic tissue that is reversibly stretchable to at least 120% of its non-stretched size.

8. A cosmetic method for figure shaping of a body part of a user's body, the method comprising the steps of:
- having the body part covered with a watertight foil element, wherein the watertight foil element is a foil item of clothing;
- having the body part wrapped by a separate elastic element, the elastic element enveloping the watertight foil, and the elastic element being under strain and thereby compressing tissue of the body part; and
- instructing the person to take exercise or to rest for a certain amount of time.

9. The method according to claim 8, including the step of applying a cosmetically effective substance to the body part prior to having the body part covered with the watertight foil.

10. The method according to claim 8 or 9, wherein the watertight foil and/or the elastic element are chosen to have a shape of the body part and to be shaped to fit on the body part.

11. A kit of parts comprising at least one device according to any one of claims 1-7 and further comprising written instructions to carry out the method according to any one of claims 8-10.

## Patentansprüche

1. Vorrichtung zur Figurformung, zur Pflege menschlichen Gewebes und/oder zur Behandlung menschlichen Gewebeseines Körperteils eines Körpers eines Benutzers, wobei die Vorrichtung aufweist:
- ein wasserdichtes Folienelement, das gestaltet ist, einen spezifischen Teil des Körpers des Benutzers zu bedecken; und
- ein elastisches Element,
wobei das elastische Element so gestaltet ist, dass das wasserdichte Folienelement zu bedecken, wenn es den Körperteil umhüllt, und dabei gedehnt zu werden, und
wobei das elastische Element separat vom wasserdichten Folienelement ausgebildet ist,
dadurch charakterisiert, dass das wasserdichte Folienelement ein Folienkleidungsstück ist.

2. Vorrichtung gemäss Anspruch 1, wobei das elastische Element mindestens ein elastisches Kleidungsstück bildet, und wobei das Folienkleidungstück mindestens alle diejenigen Körperteile des Benutzers bedeckt, die durch das elastische Kleidungsstück bedeckt werden.

3. Vorrichtung gemäss Anspruch 2, wobei sowohl das Folienkleidungsstück als auch das elastische Kleidungsstück gestaltet sind, um mindestens die Oberschenkel und den Unterleib eines menschlichen Körpers zu bedecken.

4. Vorrichtung gemäss einem der vorangehenden Ansprüche, wobei das elastische Element als elastische Hosen, elastischer Strumpf, elastische Shorts, elastischer Hülse zum teilweisen Umhüllen eines Beins, elastischer Ärmel, elastische Jacke, elastischer Pullover oder elastischer Anzug ausgebildet ist.

5. Vorrichtung gemäss einem der vorangehenden Ansprüche, aufweisend eine wirksame Substanz zum Applizieren auf die Haut des Benutzers vor dem Anlegen des wasserdichten Folienelements.

6. Vorrichtung nach einem der vorangehenden Ansprüche, wobei die Dicke des Folienelements höchstens 50 µm, vorzugsweise höchstens 30 µm, zum Beispiel höchstens 20 µm beträgt.

7. Vorrichtung nach einem der vorangehenden Ansprüche, wobei das elastische Element aus einem elastischen Gewebe gemacht ist, das reversibel auf mindestens 120% seiner nicht gedehnten Grösse dehnbar ist.

8. Verfahren zum Formen der Figur eines menschlichen Körpers, aufweisend die Verfahrensschritte:
- Bedecken des Körperteils mit wasserdichter Folie, wobei das wasserdichte Folienelement ein Folienkleidungsstück ist;
- Umhüllen des Körperteils mit einem separaten elastischen Element, wobei das elastische Element die wasserdichte Folie einhüllt, und wobei das elastische Element unter Spannung steht und dadurch Gewebe des Körperteils komprimiert; und
- Instruieren der Person, sich zu bewegen oder für eine gewisse Zeit zu ruhen.

9. Verfahren nach Anspruch 8, mit den zusätzlichen Schritt des Applizierens einer wirksamen Substanz auf den Körperteil vor dem Bedecken mit der wasserdichten Folie.

10. Verfahren nach Anspruch 8 oder 9, wobei die wasserdichte Folie und/oder das elastische Element die Form des Körperteils haben und auf den Körperteil passen.

11. Set von Teilen mit mindestens einer Vorrichtung nach einem der Ansprüche 1-7 und eine schriftliche Anleitung das Verfahren nach einem der Ansprüche 8-10 durchzuführen.

## Revendications

1. Dispositif pour façonner la silhouette et/ou prendre soin d'un tissu humain et/ou traiter un tissu humain d'une partie du corps d'un utilisateur, le dispositif comprenant :
un élément en feuille étanche à l'eau configuré pour couvrir une partie particulière du corps de l'utilisateur et
un élément élastique,
l'élément élastique étant configuré pour envelopper l'élément en feuille étanche à l'eau lorsqu'il entoure la partie du corps, et pour ainsi étant tendu,
l'élément élastique étant séparé de l'élément en feuille étanche à l'eau,
**caractérisé en ce que**
l'élément en feuille étanche à l'eau est une pièce de vêtement en feuille.

2. Dispositif selon la revendication 1, dans lequel l'élément élastique forme au moins une pièce de vêtement élastique, la pièce de vêtement en feuille couvrant au moins toutes les parties du corps de l'utilisateur qui sont couvertes par la pièce de vêtement élastique.

3. Dispositif selon la revendication 2, dans lequel la pièce de vêtement en feuille et la pièce de vêtement élastique sont toutes deux configurées pour couvrir au moins les cuisses et l'abdomen du corps humain.

4. Dispositif selon l'une quelconque des revendications précédentes, dans lequel l'élément élastique est configuré pour former des pantalons élastiques, des chaussettes élastiques, un caleçon élastique, un tube élastique destiné à être placé autour d'une partie d'une jambe, un manchon élastique destiné à être placé autour d'une partie d'un bras, une jaquette élastique, un pull élastique ou un costume élastique.

5. Dispositif selon l'une quelconque des revendications précédentes, comprenant une substance active destinée à être appliquée sur la peau de l'utilisateur avant d'y placer l'élément en feuille étanche à l'eau.

6. Dispositif selon l'une quelconque des revendications précédentes, dans lequel l'épaisseur de l'élément en feuille n'est pas supérieure à 50 µm, de préférence n'est pas supérieure à 30 µm et par exemple n'est pas supérieure à 20 µm.

7. Dispositif selon l'une quelconque des revendications précédentes, dans lequel l'élément élastique est constitué d'un tissu élastique étirable de manière réversible à au moins 120 % de sa taille non étirée.

8. Procédé cosmétique de façonnage de la silhouette d'une partie du corps d'un utilisateur, le procédé comportant les étapes qui consistent à :
recouvrir la partie du corps par un élément en feuille étanche à l'eau, l'élément en feuille étanche à l'eau étant une pièce de vêtement en feuille,
entourer la partie du corps par un élément élastique séparé, l'élément élastique enveloppant la feuille étanche à l'eau et l'élément élastique étant sous contrainte pour ainsi comprimer le tissu de la partie du corps et
aviser la personne à faire de l'exercice ou à se reposer pendant une certaine durée.

9. Procédé selon la revendication 8, comprenant l'étape qui consiste à appliquer une substance à action cosmétique sur la partie du corps avant de recouvrir la partie du corps par la feuille étanche à l'eau.

10. Procédé selon les revendications 8 ou 9, dans lequel la feuille étanche à l'eau et/ou l'élément élastique sont sélectionnés de manière à présenter la forme de la partie du corps et à être ajuster sur la partie du corps.

11. Trousse de pièce comprenant au moins un dispositif selon l'une quelconque des revendications 1 à 7 et comprenant en outre les instructions écrites permettant de mettre en oeuvre le procédé selon l'une quelconque des revendications 8 à 10.
